(19) Europäisches Patentamt
European Patent Office
Office européen des brevets

(11) Veröffentlichungsnummer: **0 000 693**
**B1**

(12) **EUROPÄISCHE PATENTSCHRIFT**

(21) Anmeldenummer: **78100205.0**

(22) Anmeldetag: **21.06.78**

(51) Int. Cl.$^3$: **C 07 D 265/30,**
A 61 K 31/535

(54) **Aminophenoxymethyl-2-morpholinderivate, ihre Herstellung und sie enthaltende Arzneimittel**

(30) Priorität: **27.06.77 DE 2728898**

(43) Veröffentlichungstag der Anmeldung:
**21.02.79 Patentblatt 79/04**

(45) Bekanntmachung des Hinweises auf die Patenterteilung:
**23.07.80 Patentblatt 80/15**

(84) Benannte Vertragsstaaten:
**DE FR GB NL**

(56) Entgegenhaltungen:
**US - A - 3 876 769**

(73) Patentinhaber: **Dr. Karl Thomae GmbH**
**Postfach 720**
**D - 7950 Biberach (Riss) (DE)**

(72) Erfinder: **Pieper, Helmut, Dr. Dipl.-Chem.**
**Kapellenweg 5**
**D - 7950 Biberach 1 (DE)**
**Noll, Klaus, Dr. Dipl.-Chem.**
**Im Schönblick 3**
**D - 7951 Warthausen 1 (DE)**
**Krüger, Gerd, Dr. Dipl.-Chem.**
**Ginsterhalde 30**
**D - 7950 Biberach 1 (DE)**
**Keck, Johannes, Dr. Dipl.-Chem.**
**Talfeldstrasse 27**
**D - 7950 Biberach 1 (DE)**
**Kähling, Joachim, Dr.**
**Haydnweg 8**
**D - 7950 Biberach 1 (DE)**
**Ziegler, Harald, Dr.**
**Händelstrasse 10**
**D - 7950 Biberach 1 (DE)**
**Ballhause, Helmut**
**Fohrenweg 6**
**D - 7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 693 B1

Courier Press, Leamington Spa, England.

# Aminophenoxymethyl-2-morpholinderivate, ihre Herstellung und sie enthaltende Arzneimittel

In der britischen Patentschrift 1 260 886 wird unter die Verbindung der Formel

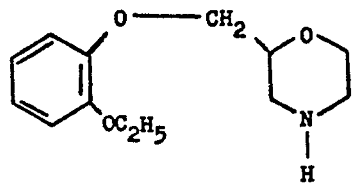

beschrieben, welche eine Wirkung auf das Zentralnervensystem aufweist.

Es wurde nun gefunden, daß die neuen Morpholinderivate der Formel

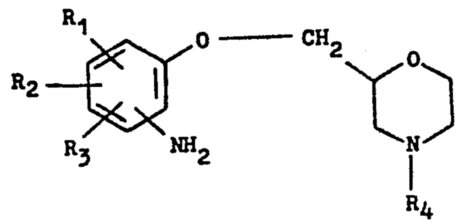

,(I)

in der

$R_1$ ein Wasserstoff- oder Halogenatom oder die Methylgruppe,
$R_2$ ein Wasserstoff- oder Halogenatom,
$R_3$ ein Wasserstoffatom oder die Nitrogruppe und
$R_4$ ein Wasserstoffatom, eine niedere Alkylgruppe, eine Carbalkoxygruppe, die Phenyl- oder Benzylgruppe bedeuten, und deren physiologisch vertägliche Säureadditionssalze mit anorganischen und organischen Säuren ebenfalls wertvolle pharmakologische Eigenschaften aufweisen, nämlich bei einer größeren therapeutischen Breite eine Wirkung auf das Zentralnervensystem, insbesondere antidepressive, antikonvulsive und/oder sedierend-muskelrelaxierende oder anxiolytische Wirkungen.

Unter dem bei der Definition der Reste $R_1$ und $R_2$ erwähnten Ausdruck "Halogenatom" ist insbesondere ein Fluor-, Chlor- oder Bromatom, unter dem bei der Definition des Restes $R_4$ erwähnten Ausdruck "niedere Alkylgruppe" insbesondere eine Alkylgruppe mit 1 bis 4 Kohlenstoffatomen" und unter "Carbalkoxygruppe" eine Carbalkoxygruppe mit insgesamt 2 bis 4 Kohlenstoffatomen zu verstehen.

Gegenstand der Erfindung sind somit vor allem die neuen Morpholinderivate der obigen allgemeinen Formel I, in der

$R_1$ ein Wasserstoff-, Fluor-, Chlor- oder Bromatom oder die Methylgruppe,
$R_2$ ein Wasserstoff-, Fluor-, Chlor- oder Bromatom,
$R_3$ ein Wasserstoffatom oder die Nitrogruppe
und
$R_4$ ein Wasserstoffatom, eine Alkylgruppe mit 1 bis 4 Kohlenstoffatomen, eine Carbalkoxygruppe mit insgesamt 2 bis 4 Kohlenstoffatomen, die Phenyl- oder Benzylgruppe bedeuten, deren physiologisch verträgliche Säureadditionssalze mit anorganischen und organischen Säuren, diese sie enthaltende Arzneimittel und Verfahren zu ihrer Herstellung.

Für die bei der Definition des Restes $R_4$ erwähnten Bedeutungen kommt somit insbesondere die des Wasserstoffatoms, der Methyl-, Äthyl-, Propyl-, Isopropyl-, n-Butyl-, Isobutyl-, sec.Butyl-, tert.Butyl-Carbomethoxy-, Carbäthoxy-, Carbisopropoxy-, Carbopropoxy-, Phenyl- oder Benzylgruppe in Betracht.

Bevorzugte Morpholin-Derivate der vorliegenden Erfindung sind jedoch die 2-Amino- und 4-Aminoverbindungen der obigen allgemeinen Formel I. Besondere bevorzugte Verbindungen der vorstehend erwähnten allgemeinen Formel I sind hierbei diejenigen, in denen einer der Reste
$R_1$ oder $R_2$ in 3- oder 5-Stellung ein Bromatom
und der andere der Reste $R_1$ oder $R_2$ in 3- oder 5-Stellung ein Wasserstoff- oder Bromatom,
$R_3$ ein Wasserstoffatom und
$R_4$ ein Wasserstoffatom, eine Alkylgruppe mit 1 bis 4 Kohlenstoffatomen oder die Carbäthoxygruppe bedeuten.

Erfindungsgemäß werden die neuen Morpholinderivate der Formel I nach folgenden Verfahren erhalten:

a) Reduktion einer Nitroverbindung der Formel

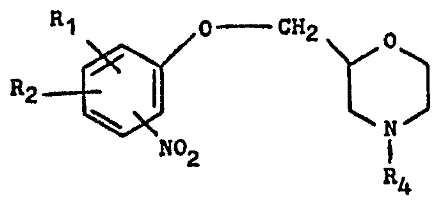

, (II)

in der

$R_1$, $R_2$ und $R_4$ wie eingangs definiert sind.

Die Reduktion wird zweckmäßigerweise in einem Lösungsmittel wie Methanol, Äthanol, Wasser/Äthanol, Äthylacetat, Eisessig oder Wasser/Salzsäure in Gegenwart eines Elektronendonators wie nascierender Wasserstoff, z.B. Zink/Salzsäure oder Eisen/Schwefelsäure, wie katalytisch angeregter Wasserstoff, z.B. Wasserstoff in Gegenwart eines Hydrierungskatalysators wie Raney-Nickel, Palladium/Kohle oder Platin, oder mit Zinn-II-chlorid/Salzsäure oder Eisen-II-sulfat/Natronlauge bei Temperaturen zwischen 0 und 100°C, vorzugsweise jedoch bei Temperaturen zwischen 0 und 80°C, durchgeführt.

Führt man die Reduktion mit einer Verbindung der allgemeinen Formel II durch, in der $R_1$ und/oder $R_2$ ein Chlor- oder Bromatom und/oder $R_4$ die Benzylgruppe darstellt, so können diese Reste bei der Reduktion mit katalytisch angeregtem Wasserstoff ganz oder teilweise während der Umsetzung durch Wasserstoffatome ersetzt werden.

b) Abspaltung einer Schutzgruppe von einer Verbindung der Formel

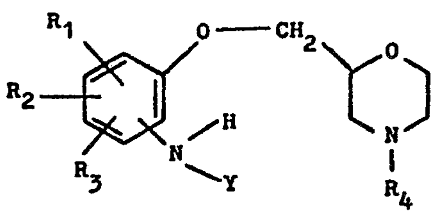

, (III)

in der

$R_1$ bis $R_4$ wie eingangs definiert sind und

Y eine Schutzgruppe für eine Aminogruppe wie die Benzyl-, Formyl-, Acetyl-, Benzoyl- oder Carbäthoxygruppe darstellt.

Stellt die Schutzgruppe Y eine Acyl- oder Kohlensäureestergruppe dar, so erfolgt die Abspaltung der Schutzgruppe hydrolytisch, vorzugsweise in einem geeigneten Lösungsmittel wie Methanol, Methanol/Wasser, Äthanol, Isopropanol oder Dioxan/Wasser in Gegenwart einer Säure wie Salzsäure oder in Gegenwart einer Base wie Natronlauge, Kalilauge oder Ammoniak bei Temperaturen zwischen 50 und 120°C, vorzugsweise jedoch bei der Siedetemperatur des Reaktionsgemisches. Bedeutet hierbei in einer Verbindung der allgemeinen Formel III $R_4$ eine Carbalkoxygruppe mit 2 bis 4 Kohlenstoffatomen, so wird diese bei der Hydrolyse durch ein Wasserstoffatom ersetzt.

Stellt die Schutzgruppe Y eine Benzylgruppe dar, so erfolgt die Abspaltung dieser Schutzgruppe hydrogenolytisch, vorzugsweise in einem geeigneten Lösungsmittel wie Methanol, Äthylacetat oder Eisessig, mit Wasserstoff in Gegenwart eines Hydrierungs-katalysators wie Raney-Nickel oder Palladium/Kohle bei Temperaturen zwischen 0 und 100°C, vorzugsweise jedoch bei Temperaturen zwischen 20 und 50°C und bei einem Wasserstoffdruck von 1—7 Atmosphären. Bedeutet hierbei in einer Verbindung der allgemeinen Formel III $R_1$ und/oder $R_2$ ein Chlor- oder Bromatom und/oder $R_4$ die Benzylgruppe, so können diese bei der Hydrogenolyse jeweils durch ein Wasserstoffatom ersetzt werden.

Eine erfindungsgemäß erhaltene Verbindung der allgemeinen Formel I, in der $R_3$ und $R_4$ wie eingangs definiert sind und $R_1$ und/oder $R_2$ ein Wasserstoffatom darstellen, kann anschließend gewünschtenfalls mittels Halogenierung in eine entsprechende Chlor- oder Bromverbindung der allgemeinen Formel I übergeführt werden

und/oder eine erhaltene Verbindung der allgemeinen Formel I, in der $R_4$ einen Aralkylrest wie die Benzylgruppe darstellt, kann anschließend mittels eines Halogen-ameisensäureesters in eine Verbindung der allgemeinen Formel I, in der $R_4$ eine Carbalkoxygruppe darstellt, übergeführt werden

und/oder eine erhaltene Verbindung der allgemeinen Formel I, in der $R_4$ eine Carbalkoxygruppe darstellt, kann anschließend mittels Hydrolyse in eine Verbindung der allgemeinen Formel I, in der $R_4$ ein Wasserstoffatom darstellt, übergeführt werden.

Die anschließende Halogenierung wird beispielsweise mit Chlor, Brom, Sulfurylchlorid oder Tribromphenolbrom, vorzugsweise in einem Lösungsmittel, z.B. in 50—100%iger Essigsäure, in Chloroform oder in Methylenchlorid, oder mit Phenyljoddichlorid in Tetrahydrofuran und in Gegenwart einer tertiären organischen Base wie Triäthylamin oder Pyridin, und zweckmäßigerweise bei Temperaturen zwischen —20 und 50°C durchgeführt. Pro Mol einer eingesetzten Verbindung der allgemeinen Formel I, welche als Base oder auch als Salz, z.B. als Mono- oder Dihydrochlorid eingesetzt werden kann, werden zweckmäßigerweise ein Mol bzw. zwei Mol an Halogenierungsmittel oder auch ein Überschuß von beispielsweise bis zu 10 Mol, vorzugsweise jedoch ein Überschuß bis zu 10% des eingesetzten Halogenierungsmittels, verwendet.

Die anschließende, Umsetzung mit einem Halogen-ameisensäureester z.B. mit Chlorameisensäuremethylester, Chlorameisensäureäthylester, Chlorameisensäurepropylester oder Chlorameisensäureisopropylester, wird zweckmäßigerweise in einem Lösungsmittel wie Methylenchlorid, Chloroform, Äthylenchlorid, Toluol oder Tetrachloräthylen bei Temperaturen zwischen 0 und 120°C, vorzugsweise jedoch bei der Siedetemperatur des verwendeten Lösungsmittels, durchgeführt.

Die anschließende Hydrolyse wird vorzugsweise in Gegenwart eine Säure wie Salzsäure oder einer Base wie Natronlauge oder Kalilauge zweckmäßigerweise in einem geeigneten Lösungsmittel wie Methanol, Methanol/Wasser, Äthanol, Isopropanol oder Dioxan/Wasser bei Temperaturen zwischen 50 und 120°C, vorzugsweise jedoch bei der Siedetemperatur des Reaktionsgemisches, durchgeführt.

Ferner können die erfindungsgemäß erhaltenen Verbindungen der allgemeinen Formel I gewünschtenfalls mit anorganischen oder organischen Säuren in ihre physiologisch verträglichen Säureadditionssalze mit ein oder zwei Äquivalenten der betreffenden Säure übergeführt werden. Hierbei haben sich beispielsweise Salzsäure, Bromwasserstoffsäure, Schwefelsäure, Phosphorsäure, Milchsäure, Zitronensäure, Weinsäure, Maleinsäure oder Fumarsäure als geeignet erwiesen.

Die als Ausgangsstoffe verwendeten Verbindungen der allgemeinen Formeln II und III erhält man nach an sich bekannten Verfahren, beispielsweise durch Umsetzung eines entsprechenden Phenols mit Epichlorhydrin in Gegenwart von Natrium- oder Kaliumhydroxid; das so erhaltene Epoxiderivat wird durch Umsetzung mit einem entsprechenden Amin und anschließende Cyclysierung mit Thionylchlorid in Dimethylformamid zu dem entsprechenden Morpholinderivat übergeführt.

Wie bereits eingangs erwähnt, weisen die neuen Morpholinderivate der allgemeinen Formel I und deren physiologisch verträgliche Säureadditionssalze mit anorganischen und organischen Säuren wertvolle pharmakologische Eigenschaften auf, nämlich eine Wirkung auf das Zentralnervensystem, insbesondere jedoch antidepressive, antikonvulsive und/oder sedierend-muskelrelaxierende oder anxiolytische Wirkungen.

Beispielsweise wurden die Verbindungen

A = 2-(2-Amino-3,5-dibrom-phenoxy-methylen)-morpholin-hydrochlorid,
B = 2-(4-Amino-3,5-dibrom-phenoxy-methylen)-4-isopropyl-morpholin-hydrochlorid,
C = 2-(4-Amino-3,5-dibrom-phenoxy-methylen)-morpholin-hydrochlorid,
D = 2-(2-Amino-3,5-dibrom-phenoxy-methylen)-4-tert.butyl-morpholin-hydrochlorid und
E = 2-(2-Amino-5-brom-4-chlor-phenoxy-methylen)-4-isopropyl-morpholin-dihydrochlorid
im Vergleich zu
F = 2-(2-Äthoxy-phenoxy-methylen)-morpholin-hydrochlorid

auf ihre biologischen Wirkungen wie folgt untersucht:

1. Die *antidepressive Wirkung* wurde am Modell des Antagonismus gegen die durch Reserpin hervorgerufene Hypothermie bei Mäusen (ASKEW, B. M., Life Sci. *2,* 725, 1963) geprüft. Bei 20—26 g schweren männlichen Mäusen wurde durch 3 mg/kg Reserpin s.c. Hypothermie erzeugt. 17 Stunden später wurde die Körpertemperatur mittels eines elektrischen Schlundsondenthermometers gemessen. Anschließend wurden Gruppen von 6—10 Tieren die zu prüfenden Substanzen in der Dosis von 40 mg/kg p.o. verabreicht. Kontrollgruppen erhielten das Lösungsmittel (Aqua dest.). Die Körpertemperaturen wurden 1—4 Stunden nach Substanzverabreichung in stündlichem Abstand gemessen. Zur Auswertung wurden der maximale Wiederansteig der Körpertemperatur der Substanzgruppe mit den Temperaturen der Kontrolltiergruppen verglichen und die Temperaturunterschiede mit dem MANN-WHITNEY-U-Test auf Signifikanz geprüft.

2. Die *antikonvulsive Wirkung* der zu prüfenden Substanzen wurde am Modell der Wirkung gegen den maximalen Elektrokrampf bei Mäusen (SWINYARD, E. A., BROWN, W. C., GOODMAN, L. A., J. Pharmacol. exp. Ther *106*, 319, 1952) ermittelt. Dazu wurde bei 10 männlichen, 20—26 g schweren Mäusen ein Krampfzustand mit Wechselstrom (50 mA, 50 Hz, 0,2 sec Dauer) über mit physiologischer Kochsalz-Lösung angefeuchtete, an den Kopf der Tiere gelegte Metallelektroden erzeugt, wobei das Ausbleiben der tonischen Extensorkomponente dieses Krampfes 150 Minuten nach Substanzgabe als antikonvulsive Wirkung gewertet wurde. $ED_{50}$-Werte wurden als Dosen mit 50%iger Krampfhemmung nach LITCHFIELD und WILCOXON (J. Pharmacol. exp. Ther. *96,* 99, 1949) berechnet.

3. Die *muskelrelaxierend-sedierende Wirkung* der zu prüfenden Verbindungen wurde mit der Methode der Beeinflussung des Haltevermögens von Mäusen in rotierenden Drahtzylindern (YOUNG, D. M., LEWIS, A. H., Science *10£,* 368, 1947) geprüft. Hierbei wurden bei jeweils 10 weiblichen 20—26 g schweren Mäusen pro Dosis 90—120 Minuten nach oraler Substanzverabreichung die Anzahl der im

Versuchsverlauf aus den Zylindern fallenden Tiere festgestellt. $ED_{50}$-Werte wurden graphisch als die Dosen ermittelt, bei denen sich 50% der Tiere nicht in den Zylindern halten konnten.

4. Die *angsthemmende Wirkung* der Substanzen wurde an 160—190 g schweren, weiblichen Ratten nach dem Verfahren von J. R. VOGEL, B. BEER und D. E. CLODY (Psychopharmacologia (Berl.) *21,* 1, 1971) geprüft. Hierbei diente die Anzahl der von durstigen Ratten beim Trinken hingenommenen Elektroschocks als Maß der Angsthemmung. Tiere unter dem Ainfluß angsthemmender Substanzen nehmen mehr Elektroschocks hin als Kontrolltiere. Die zu prüfenden Substanzen wurden den Tieren eine Stunde vor Versuch in der Standarddosis von 10 mg/kg p.o., verabreicht. Die Differenzen in der Anzahl hingenommener Elektroschocks zwischen Substanztieren und Kontrolltieren wurden mit dem MANN-WHITNEY-U-Test auf statistische Signifikanz geprüft. Die mittlere Anzahl der Trinkakte betrug bei den Kontrollgruppen 2,9±0,4 pro Tier.

Die nachfolgende Tabelle enthält die Ergebnisse der Versuche 1 bis 4:

| Substanz | Versuch 1 Reserpin Maus 40 mg/kg: +°C | Versuch 2 Elektrokrampf Maus $ED_{50}$–Hemmung mg/kg | Versuch 3 Muskelrelax. + Sedierung Maus $ED_{50}$, mg/kg | Versuch 4 Angsthemmung Ratte bei 10 mg/kg |
|---|---|---|---|---|
| A | 10,7* | 26,5 | >200 | +0,6 |
| B | 8,4* | 26 | 60 | −0,2 |
| C | 7,3* | 17,5 | 21 | +1,1 |
| D | 0,6 | 150 | >200 | +5,1* |
| E | 1,0 | 200 | >200 | +5,8* |
| F | 9,8* | 42 | ~200 | +1,1 |

* Signifikanter Unterschied gegenüber Kontrollen, $p \leq 0{,}05$

5. Die *toxische Wirkung* der zu prüfenden Substanzen wurde

a) als $LD_{50}$-Wert, als Dosis bei der 50% der Tiere nach einmaliger oraler Substanzverabreichung und 14-tägiger Nachbeobachtung starben, nach dem Verfahren von LITCHFIELD und WILCOXON (J. Pharmacol. exp. Ther. *96,* 99, 1949) ermittelt. Hierbei wurden pro Dosis 5 weibliche und 5 männliche 20—26 g schwere Mäuse verwendet.

b) In orientierenden Toxizitätsprüfungen wurden 5 weiblichen 20—26 g schweren Mäusen die Substanzen mit 200, 500 oder 1,000 mg/kg oral verabreicht und die Tiere 24 Stunden nachbeobachtet:

| Substanz | Toxische Wirkung |
|---|---|
| A | $LD_{50}$: 1520 mg/kg p.o. |
| B | > 500 mg/kg p.o. (0 von 5 Tieren gestorben) |
| C | > 200 mg/kg p.o. (0 von 5 Tieren gestorben) |
| D | > 500 mg/kg p.o. (0 von 5 Tieren gestorben) |
| E | > 1 000 mg/kg p.o. (1 von 5 Tieren gestorben) |
| F | $LD_{50}$: 520 mg/kg p.o. |

Die erfindungsgemäß erhaltenen Morpholinderivate der allgemeinen Formel I und deren physiologisch verträgliche Säureadditionssalze mit anorganischen und organischen Säuren eignen sich daher

insbesondere zur Behandlung von Depressionen und von Angstzuständen und lassen sich zur pharmazeutischen Anwendung in die üblichen pharmazeutischen Zubereitungsformen wie Tabletten, Dragées, Kapseln, Suppositorien, Ampullen, Suspensionen oder Lösungen, gegebenenfalls in Kombination mit anderen Wirksubstanzen, einarbeiten. Die Einzeldosis am Menschen beträgt hierbei zweckmäßigerweise 20 bis 50 mg.

Die nachfolgenden Beispiele sollen die Erfindung näher erläutern:

BEISPIEL A

*1,2-Epoxy-3-(2-nitro-phenoxy)-propan*

Eine Mischung von 318 g o-Nitrophenol, 304 g Epichlorhydrin und 160 g festem Kaliumhydroxid in 3 l Wasser werden 16 Stunden lang bei Raumtemperatur gerührt. Nach dieser Zeit wird die Mischung 3 × mit je 1000 ml Chloroform extrahiert. Die vereinigte Chloroform-Phase wird mit Wasser gewaschen, über Natriumsulfat getrock-net und im Vakuum am Rotationsverdampfer zur Trockne eingedampft. Der ölige Rückstand wird in 500 ml Isopropanol aufgenommen. Nach Zusatz von 500 ml Petroläther tritt Kristallisation ein. Die Kristalle werden abgesaugt und mit Petroläther gewaschen. Schmelzpunkt: 45—47°C.

BEISPIEL B

*4-Benzyl-2-(2-nitro-phenoxy-methylen)-morpholin-hydrochlorid*

70 g 1,2-Epoxy-3-(2-nitro-phenoxy)-propan und 57,5 g Benzylaminoäthanol werden in 300 ml Chloroform gelöst. Nach Abdampfen des Lösungsmittels im Vakuum wird die Mischung 3 Stunden lang auf 140°C erhitzt. Nach dieser Zeit wird in 300 ml Dimethylformamid gelöst und diese Lösung auf 0°C abgekühlt. Bei dieser Temperatur gibt man unter Rühren tropfenweise 29,6 ml Thionylchlorid zu, rührt 3 Stunden lang bei Raumtemperatur und erhitzt anschließend 16 Stunden lang auf Rückflußtemperatur. Nach Abkühlen wird in Eis/Natronlauge eingerührt, wobei darauf geachtet wird, daß der pH-Wert > 7 bleibt. Es fällt ein schlecht filtrierbarer Niederschlag aus, der dreimal mit je 250 ml Chloroform extrahiert wird. Die vereinigten Chloroform-Phasen werden mit Wasser gewaschen, über Natriumsulfat getrocknet und im Vakuum zur Trockne eingeengt. Der verbleibende Rückstand wird über 1 kg Kieselgel säulenchromatographiert, wobei eine Mischung von Chloroform/Essigester = 4/1 als Elutionsmittel dient. Die vereinigten Substanz enthaltenden Eluate werden im Vakuum zur Trockne eingedampft. Der Rückstand wird in Isopropanol aufgenommen und diese Lösung mit ätherischer Salzsäure angesäuert. Es tritt Kristallisation ein. Die ausgeschiedenen Kristalle werden abgesaugt und aus Isopropanol/Äther = 1/1 umkristallisiert. Schmelzpunkt: 206—208°C.

Analog den Beispielen A und B wurden folgende Verbindungen hergestellt:

4-tert.Butyl-2-(2-nitro-phenoxy-methylen)-morpholin-hydrochlorid
Schmelzpunkt: 175—177°C

4-Methyl-2-(4-nitro-phenoxy-methylen)-morpholin-hydrochlorid
Schmelzpunkt: 216—218°C

4-Methyl-2-(2-nitro-phenoxy-methylen)-morpholin
Schaum

4-Äthyl-2-(4-nitro-phenoxy-methylen)-morpholin-hydrochlorid
Schmelzpunkt: 240—242°C

4-Isopropyl-2-(4-nitro-phenoxy-methylen)-morpholin-hydrochlorid
Schmelzpunkt: 195—197°C

2-(4-Nitro-phenoxy-methylen)-4-methyl-morpholin
Schmelzpunkt: 105—107°C

4-tert.Butyl-2-(4-nitro-phenoxy-methylen)-morpholin-hydrochlorid
Schmelzpunkt: 201—203°C

4-Isopropyl-2-(2-nitro-phenoxy-methylen)-morpholin-hydrochlorid
Schmelzpunkt: 187—189°C

4-Benzyl-2-(5-methyl-2-nitro-phenoxy-methylen)-morpholin-hydrochlorid
Schmelzpunkt: 192—194°C

4-Benzyl-2-(2-nitro-phenoxy-methylen)-morpholin-hydrochlorid
Schmelzpunkt: 206—208°C

2-(4-Chlor-2-nitro-phenoxy-methylen)-4-isopropyl-morpholin-hydrochlorid
Schmelzpunkt: 208—210°C

4-Benzyl-2-(4-chlor-2-nitro-phenoxy-methylen)-morpholin-hydrochlorid
Schmelzpunkt: 218—220°C

2-(2-Chlor-4-nitro-phenoxy-methylen)-4-isopropyl-morpholin-hydrochlorid
Schmelzpunkt: 205—206°C

4-Benzyl-2-(2-chlor-4-nitro-phenoxy-methylen)-morpholin-hydrochlorid
Schmelzpunkt: 198—200°C

4-Benzyl-2-(5-methyl-2-nitro-phenoxy-methylen)-morpholin-hydrochlorid
Schmelzpunkt: 198—200°C

4-Benzyl-2-(3-methyl-4-nitro-phenoxy-methylen)-morpholin-hydrochlorid
Schmelzpunkt: 217—218°C

Die folgeden Verbindungen wurden durch Umsetzung der entsprechenden Benzylverbindung mit Carbäthoxychlorid (siehe Beispiel 6) hergestellt:
4-Carbäthoxy-2-(2-chlor-4-nitro-phenoxy-methylen)-morpholin
Schmelzpunkt: 106—108°C
4-Carbäthoxy-2-(4-chlor-2-nitro-phenoxy-methylen)-morpholin
Schmelzpunkt: 91—93°C
4-Carbäthoxy-2-(3-methyl-4-nitro-phenoxy-methylen)-morpholin
Schmelzpunkt: 88—91°C

BEISPIEL C

*3-(4-Acetamino-phenoxy)-1,2-epoxy-propan*

Eine Mischung aus 178 g 4-Hydroxy-acetanilid, 166 g Epichlorhydrin und 52 g festem Natriumhydroxid in 1,2 l Wasser wird 20 Stunden lang bei Raumtemperatur gerührt. Der feste Niederschlag wird abgesaugt, mit Wasser gewaschen und aus Isopropanol kristallisiert.
Schmelzpunkt: 116—118°C.

BEISPIEL D

*2-(4-Acetamino-phenoxy-methylen)-4-benzyl-morpholin*

51 g 3-(4-Acetamino-phenoxy)-1,2-epoxy-propan und 37,8 g Benzylaminoäthanol werden in 250 ml Chloroform gelöst. Nach Abdampfen des Lösungsmittels im Vakuum wird die Mischung 3 Stunden lang auf 140°C erhitzt. Nach dieser Zeit wird in 200 ml Dimethylformamid gelöst und diese Lösung auf 0°C abgekühlt. Bei dieser Temperatur gibt man unter Rühren tropfenweise 19,6 ml Thionylchlorid zu, rührt 3 Stunden lang bei Raumtemperatur und erhitzt anschliessend 16 Stunden lang auf Rückflußtemperatur. Nach Abkühlen wird in Eis/Natronlauge eingerührt, wobei darauf geachtet wird, daß der pH-Wert > 7 bleibt. Es fällt ein schlecht filtrierbarer Niederschlag aus, der dreimal mit je 200 ml Chloroform extrahiert wird. Die vereinigte Chloroform-Phase wird mit Wasser gewaschen, über Natriumsulfat getrocknet und im Vakuum zur Trockne eingeengt. Der verbleibende Rückstand wird über 800 g Kieselgel säulenchromatographiert, wobei Essigester als Elutionsmittel dient. Die vereinigten Substanz enthaltenden Eluate werden im Vakuum zur Trockne eingedampft. Der Rückstand wird in Toluol aufgenommen und durch Zusatz von Petroläther zur Kristallisation gebracht. Die Kristalle werden abgesaugt und mit Petroläther gewaschen.
Schmelzpunkt: 120—122°C.

Analog den Beispielen C und D wurden folgende Substanzen hergestellt:
2-(4-Acetamino-phenoxy-methylen)-4-benzyl-morpholin-hydrochlorid
Schaum
2-(2-Acetamino-4-nitro-phenoxy-methylen)-4-benzyl-morpholin Öl
2-(2-Acetamino-4-nitro-phenoxy-methylen)-4-isopropyl-morpholin Öl

BEISPIEL 1

*2-(2-Amino-phenoxy-methylen)-morpholin-hydrochlorid*

25 g 4-Benzyl-2-(2-nitro-phenoxy-methylen)-morpholin-hydrochlorid werden in 450 ml Methanol gelöst und nach Zusatz von 2 g 10%iger Palladiumkohle bei 5 at Druck und bei Raumtemperatur hydriert, bis die theoretische Menge Wasserstoff aufgenommen ist. Nach Entfernen des Katalysators wird die Methanol-Lösung im Vakuum zur Trockne eingeengt, der Rückstand in heißem Äthanol gelöst und die Lösung mit ätherischer Salzsäure versetzt. Beim Stehenlassen tritt Kristallisation ein. Die Kristalle werden abgesaugt und mit einer Mischung aus Äthanol und Äther gewaschen.
Schmelzpunkt: 126—130°C.

BEISPIEL 2

*2-(2-Amino-phenoxy-methylen)-4-tert.butyl-morpholin-hydrochlorid*

16 g 4-tert.Butyl-2-(2-nitro-phenoxy-methylen)-morpholin-hydrochlorid werden in 150 ml Methanol gelöst und nach Zusatz von 1 g 10%iger Palladiumkohle bei 5 at Druck und bei Raumtemperatur hydriert, bis die theoretische Menge Wasserstoff aufgenommen ist. Nach Entfernen des Katalysators wird die Methanol-Lösung im Vakuum zur Trockne eingeengt und der Rückstand aus Äthanol kristallisiert.
Schmelzpunkt: 224—226°C (Zers.).

BEISPIEL 3

*4-Äthoxycarbonyl-2-(4-amino-2-chlor-phenoxy-methylen)-morpholin-hydrochlorid*

12 g 4-Äthoxycarbonyl-2-(2-chlor-4-nitro-phenoxy-methylen)-morpholin werden in 200 ml Äthanol gelöst. Unter Kühlung mit Eis und unter Rühren tropft man 150 ml konz. Salzsäure zu und gibt danach unter weiterer Kühlung während 1,5 Stunden portionsweise 75 g Zinn-II-chlorid zu. Nach beendeter Zugabe gießt man auf Eis, stellt mit 10 n Natronlauge alkalisch und extrahiert 3 mal mit je 200 ml Chloroform. Die vereinigten Chloroform-Extrakte werden mit Wasser gewaschen, über Natriumsulfat getrocknet und im Vakuum zur Trockne eingedampft. Der Rückstand wird in Isopropanol gelöst und die Isopropanol-Lösung mit ätherischer Salzsäure angesäuert. Es tritt Kristallisation ein. Die ausgeschiedenen Kristalle werden abgesaugt und mit Isopropanol gewaschen.

Schmelzpunkt: 116—117°C (Zers.).

BEISPIEL 4

*2-(2-Amino-4-nitro-phenoxy-methylen)-4-isopropyl-morpholin-dihydrochlorid*

17 g 2-(2-Acetamino-4-nitro-phenoxy-methylen)-4-isopropyl-morpholin werden in 200 ml Äthanol gelöst und nach Zugabe einer Lösung von 5 g Natriumhydroxid in 50 ml Wasser 2 Stunden lang am Rückfluß gekocht. Die Lösung wird zur Entfernung des Alkohols eingeengt, der Rückstand wird zwischen Wasser und Chloroform verteilt; der Chloroformextrakt wird getrocknet und zur Trockne eingeengt. Der Rückstand wird in Isopropanol gelöst und mit äthanolischer Salzsäure angesäuert, worauf 2-(2-Amino-4-nitro-phenoxy-methylen)-4-isopropyl-morpholin-dihydrochlorid auskristallisiert; es wird abgesaugt mit wenig Isopropanol gewaschen und getrocknet.

Schmelzpunkt: 230—233°C (Zers.).

BEISPIEL 5

*2-(2-Amino-3,5-dibrom-phenoxy-methylen)-morpholin-hydrochlorid*

9 g 2-(2-Amino-phenoxy-methylen)-morpholin-hydrochlorid werden in 100 ml Eisessig gelöst. Unter Rühren tropft man hierzu eine Lösung von 3,7 ml Brom in 20 ml Eisessig. Nach beendeter Zugabe gießt man in Eiswasser, stellt mit 10 n Natronlauge alkalisch und extrahiert 3 mal mit je 200 ml Chloroform. Die vereinigten Chloroform-Extrakte werden mit Wasser gewaschen, über Natriumsulfat getrocknet und im Vakuum zur Trockne eingeengt. Der Rückstand wird in Äthanol gelöst und mit ätherischer Salzsäure angesäuert. Der auskristallisierende Niederschlag wird abgesaugt und das Äthanol kristallisiert.

Schmelzpunkt: 207—209°C.

BEISPIEL 6

*2-(2-Amino-3,5-dibrom-4-nitro-phenoxy-methylen)-4-carbäthoxy-morpholin*

16 g 2-(2-Amino-3,5-dibrom-4-nitro-phenoxy-methylen)-4-benzyl-morpholin werden in 350 ml Toluol gelöst und nach Zugabe von 5 ml Carbäthoxychlorid 4 Stunden am Rückfluß gekocht. Das Reaktionsgemisch wird nach ca. 15-stündigem Stehen bei Raumtemperatur im Vakuum eingeengt, der Rückstand mit Isopropanol verrieben, abgesaugt und aus Isopropanol umkristallisiert, wobei 2-(2-Amino-3,5-dibrom-4-nitro-phenoxy-methylen)-4-carbäthoxy-morpholin vom.

Schmelzpunkt: 105—107°C erhalten wird.

BEISPIEL 7

*2-(4-Amino-2-chlor-phenoxy-methylen)-morpholin-hydrochlorid*

9 g 4-Äthoxycarbonyl-2-(4-amino-2-chlor-phenoxy-methylen)-morpholin-hydrochlorid werden in 100 ml Methanol gelöst. Nach Zusatz von 10 g festem Natriumhydroxid wird 3 Stunden lang auf Rückfluß-temperatur erhitzt. Die Lösung wird anschließend im Vakuum zur Trockne eingeengt und der Rückstand zwischen Wasser und 100 ml Chloroform verteilt. Die wässrige Phase wird noch 2 mal mit je 100 ml Chloroform extrahiert. Die vereinigten Chloroform-Extrakte werden mit Wasser gewaschen, über Natriumsulfat getrocknet und im Vakuum zur Trockne eingedampft. Der Rückstand wird in Isopropanol gelöst. Die Lösung wird mit ätherischer Salzsäure angesäuert. Der auskristallisierende Niederschlag wird abgesaugt und aus Äthanol kristallisiert.

Schmelzpunkt: 271—274°C.

BEISPIEL 8

*2-(4-Amino-phenoxy-methylen)-4-methyl-morpholin-hydrochlorid*

Hergestellt aus 4-Methyl-2-(4-nitro-phenoxy-methylen)-morpholin durch katalytische Hydrierung analog Beispiel 2.

Schmelzpunkt: 179—182°C.

BEISPIEL 9

*2-(2-Amino-phenoxy-methylen)-4-methyl-morpholin-dihydrochlorid*

Hergestellt aus 4-Methyl-2-(2-nitro-phenoxy-methylen)-morpholin durch katalytische Hydrierung analog Beispiel 2.

Schmelzpunkt: 262—265°C (Zers.).

BEISPIEL 10

*2-(4-Amino-phenoxy-methylen)-morpholin-dihydrochlorid*

Hergestellt aus 2-(4-Amino-phenoxy-methylen)-4-benzyl-morpholin-dihydrochlorid durch katalytische Hydrierung analog Beispiel 1.

Schmelzpunkt: 250—254°C.

BEISPIEL 11

*4-Äthyl-2-(4-amino-phenoxy-methylen)-morpholin-hydrochlorid*

Hergestellt aus 4-Äthyl-2-(4-nitro-phenoxy-methylen)-morpholin-hydrochlorid durch katalytische Hydrierung analog Beispiel 2.

Schmelzpunkt: 199—200°C.

BEISPIEL 12

*2-(4-Amino-phenoxy-methylen)-4-isopropyl-morpholin-hydrochlorid*

Hergestellt aus 4-Isopropyl-2-(4-nitro-phenoxy-methylen)-morpholin-hydrochlorid durch katalytische Hydrierung analog Beispiel 2.

Schmelzpunkt: 179—181°C.

BEISPIEL 13

*2-(4-Amino-phenoxy-methylen)-4-phenyl-morpholin-hydrochlorid*

Hergestellt aus 2-(4-Nitro-phenoxy-methylen)-4-phenyl-morpholin durch katalytische Hydrierung analog Beispiel 2.

Schmelzpunkt: 210—215°C (Zers.).

BEISPIEL 14

*2-(4-Amino-phenoxy-methylen)-4-tert.butyl-morpholin-hydrochlorid*

Hergestellt aus 4-tert.Butyl-2-(4-nitro-phenoxy-methylen)-morpholin-hydrochlorid durch katalytische Hydrierung analog Beispiel 2.

Schmelzpunkt: 155—157°C.

BEISPIEL 15

*2-(2-Amino-phenoxy-methylen)-4-isopropyl-morpholin-hydrochlorid*

Hergestellt aus 4-Isopropyl-2-(2-nitro-phenoxy-methylen)-morpholin-hydrochlorid durch katalytische Hydrierung analog Beispiel 2.

Schmelzpunkt: 165—167°C.

BEISPIEL 16

*2-(2-Amino-5-methyl-phenoxy-methylen)-morpholin*

Hergestellt aus 4-Benzyl-2-(5-methyl-2-nitro-phenoxy-methylen)-morpholin-hydrochlorid durch katalytische Hydrierung analog Beispiel 1.

Schmelzpunkt: 108—110°C.

BEISPIEL 17

*2-(2-Amino-phenoxy-methylen)-4-benzyl-morpholin-dihydrochlorid*

Hergestellt aus 4-Benzyl-2-(2-nitro-phenoxy-methylen)-morpholin-hydrochlorid durch Reduktion mit Zinn-II-chlorid analog Beispiel 3.

Schmelzpunkt: 248—253°C.

BEISPIEL 18

*2-(2-Amino-4-chlor-phenoxy-methylen)-4-isopropyl-morpholin-dihydrochlorid*

Hergestellt aus 2-(4-Chlor-2-nitro-phenoxy-methylen)-4-isopropyl-morpholin-hydrochlorid durch Reduktion mit Zinn-II-chlorid analog Beispiel 3.

Schmelzpunkt: 248—252°C.

BEISPIEL 19

*2-(2-Amino-4-chlor-phenoxy-methylen)-4-benzyl-morpholin-hydrochlorid*

Hergestellt aus 4-Benzyl-2-(4-chlor-2-nitro-phenoxy-methylen)-morpholin-hydrochlorid durch Reduktion mit Zinn-II-chlorid analog Beispiel 3.

Schmelzpunkt: 262—266°C.

BEISPIEL 20

*2-(2-Amino-4-chlor-phenoxy-methylen)-4-carbäthoxy-morpholin*

Hergestellt aus 4-Carbäthoxy-2-(4-chlor-2-nitro-phenoxy-methylen)-morpholin durch Reduktion mit Zinn-II-chlorid analog Beispiel 3.

Schmelzpunkt: 110—112°C.

BEISPIEL 21

*2-(4-Amino-2-chlor-phenoxy-methylen)-4-isopropyl-morpholin-hydrochlorid*

Hergestellt aus 2-(2-Chlor-4-nitro-phenoxy-methylen)-4-isopropyl-morpholin-hydrochlorid durch Reduktion mit Zinn-II-chlorid analog Beispiel 3.

Schaum, Strukturbeweis durch NMR-Spektrum ($CDCl_3$):

6,3 —6,8 ppm Multiplett (3 arom. H)

5,2 —6,2 ppm Singulett (3H: $NH_2$ + $HCl$)

3,8 —3,95 ppm Dublett (2H: 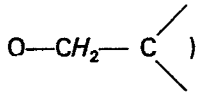 )

1,25—1,35 ppm Dublett (6H: 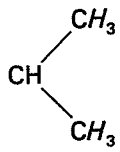 )

BEISPIEL 22

*2-(4-Amino-2-chlor-phenoxy-methylen)-4-benzyl-morpholin-hydrochlorid*

Hergestellt aus 4-Benzyl-2-(2-chlor-4-nitro-phenoxy-methylen)-morpholin-hydrochlorid durch Reduktion mit Zinn-II-chlorid analog Beispiel 3.

Schmelzpunkt: 229—230°C.

BEISPIEL 23

*2-(2-Amino-5-methyl-phenoxy-methylen)-4-benzyl-morpholin-hydrochlorid*

Hergestellt aus 4-Benzyl-2-(5-methyl-2-nitro-phenoxy-methylen)-morpholin-hydrochlorid durch Reduktion mit Zinn-II-chlorid analog Beispiel 3.

Schmelzpunkt: 191—193°C.

BEISPIEL 24

*2-(4-Amino-3-methyl-phenoxy-methylen)-4-benzyl-morpholin-hydrochlorid*

Hergestellt aus 4-Benzyl-2-(3-methyl-4-nitro-phenoxy-methylen)-morpholin-hydrochlorid durch Reduktion mit Zinn-II-chlorid analog Beispiel 3.

Schmelzpunkt: 172—175°C.

BEISPIEL 25

*2-(4-Amino-3-methyl-phenoxy-methylen)-4-carbäthoxy-morpholin-hydrochlorid*

Hergestellt aus 4-Carbäthoxy-2-(3-methyl-4-nitro-phenoxy-methylen)-morpholin-hydrochlorid durch Reduktion mit Zinn-II-chlorid analog Beispiel 3.

Schmelzpunkt: 195—198°C (Zers.).

BEISPIEL 26

*2-(2-Amino-4-nitro-phenoxy-methylen)-4-benzyl-morpholin-dihydrochlorid*

Hergestellt aus 2-(2-Acetamino-4-nitro-phenoxy-methylen)-4-benzyl-morpholin und Natronlauge analog Beispiel 4.

Schmelzpunkt: 242—248°C (Zers.).

BEISPIEL 27

*2-(4-Amino-phenoxy-methylen)-4-benzyl-morpholin-dihydrochlorid*

Hergestellt aus 2-(4-Acetamino-phenoxy-methylen)-4-benzyl-morpholin und Natronlauge analog Beispiel 4.

Schaum.

| | | | |
|---|---|---|---|
| Ber.: | C 70,56 | H 7,11 | N 8,23 |
| Gef.: | 70,90 | 7,20 | 8,04 |

BEISPIEL 28

*2-(4-Amino-3,5-dibrom-phenoxy-methylen)-4-methyl-morpholin-hydrochlorid*

Hergestellt aus 2-(4-Amino-phenoxy-methylen)-4-methyl-morpholin-hydrochlorid und Brom analog Beispiel 5.

Schmelzpunkt: 211—213°C (Zers.).

BEISPIEL 29

*2-(2-Amino-3,5-dibrom-phenoxy-methylen)-4-methyl-morpholin-hydrochlorid*

Hergestellt aus 2-(2-Amino-phenoxy-methylen)-4-methyl-morpholin-hydrochlorid und Brom analog Beispiel 5.

Schmelzpunkt: 207—209°C.

BEISPIEL 30

*2-(4-Amino-3,5-dibrom-phenoxy-methylen)-4-isopropyl-morpholin-hydrochlorid*

Hergestellt aus 2-(4-Amino-phenoxy-methylen)-4-isopropyl-morpholin-hydrochlorid und Brom analog Beispiel 5.

Schmelzpunkt: 178—180°C.

BEISPIEL 31

*2-(4-Amino-3,5-dibrom-phenoxy-methylen)-morpholin-hydrochlorid*

Hergestellt aus 2-(4-Amino-phenoxy-methylen)-morpholin-dihydrochlorid und Brom analog Beispiel 5.

Schmelzpunkt: 183—185°C.

BEISPIEL 32

*2-(4-Amino-3,5-dibrom-phenoxy-methylen)-4-benzyl-morpholin-hydrochlorid*

Hergestellt aus 2-(4-Amino-phenoxy-methylen)-4-benzyl-morpholin-dihydrochlorid und Brom analog Beispiel 5.

Schmelzpunkt: 234—236°C.

BEISPIEL 33

*2-(4-Amino-3,5-dibrom-phenoxy-methylen)-4-tert.butyl-morpholin-hydrochlorid*

Hergestellt aus 2-(4-Amino-phenoxy-methylen)-4-tert.butyl-morpholin-hydrochlorid und Brom analog Beispiel 5.

Schmelzpunkt: 228—230°C (Zers.).

BEISPIEL 34

*2-(2-Amino-3,5-dibrom-phenoxy-methylen)-4-isopropyl-morpholin-hydrochlorid*

Hergestellt aus 2-(2-Amino-phenoxy-methylen)-4-isopropyl-morpholin-hydrochlorid und Brom analog Beispiel 5.

Schmelzpunkt: 115—117°C (Zers.).

BEISPIEL 35

*4-Äthyl-2-(2-amino-3,5-dibrom-phenoxy-methylen)-morpholin-hydrochorid*

Hergestellt aus 4-Äthyl-2-(2-amino-phenoxy-methylen)-morpholin-hydrochlorid und Brom analog Beispiel 5.

Schmelzpunkt: 255—257°C.

BEISPIEL 36

*2-(2-Amino-3,5-dibrom-phenoxy-methylen)-4-benzyl-morpholin-hydrochlorid*

Hergestellt aus 2-(2-Amino-phenoxy-methylen)-4-benzyl-morpholin-dihyrochlorid und Brom analog Beispiel 5.

Schmelzpunkt: 220—225°C.

BEISPIEL 37

*2-(2-Amino-3,5-dibrom-phenoxy-methylen)-4-tert.butyl-morpholin-hydrochlorid*

Hergestellt aus 2-(2-Amino-phenoxy-methylen)-4-tert.butyl-morpholin-hydrochlorid und Brom analog Beispiel 5.

Schmelzpunkt: 243—245°C (Zers.).

BEISPIEL 38

*2-(2-Amino-5-brom-4-chlor-phenoxy-methylen)-4-isopropyl-morpholin-dihydrochlorid*

Hergestellt aus 2-(2-Amino-4-chlor-phenoxy-methylen)-4-isopropyl-morpholin-dihydrochlorid und Brom analog Beispiel 5.

Schmelzpunkt: 225—228°C (Zers.).

BEISPIEL 39

*2-(2-Amino-5-brom-4-chlor-phenoxy-methylen)-4-benzyl-morpholin-hydrochlorid*

Hergestellt aus 2-(2-Amino-4-chlor-phenoxy-methylen)-4-benzyl-morpholin-dihydrochlorid und Brom analog Beispiel 5.

Schmelzpunkt: 235—237°C.

BEISPIEL 40

*2-(2-Amino-5-brom-4-chlor-phenoxy-methylen)-morpholin*

Hergestellt aus 2-(2-Amino-4-chlor-phenoxy-methylen)-morpholin-dihydrochlorid und Brom analog Beispiel 5.

Schmelzpunkt: 130—132°C.

BEISPIEL 41

*2-(4-Amino-5-brom-2-chlor-phenoxy-methylen)-4-benzyl-morpholin-dihydrochlorid*

Hergestellt aus 2-(4-Amino-2-chlor-phenoxy-methylen)-4-benzyl-morpholin-hydrochlorid und Brom analog Beispiel 5.

Schmelzpunkt: 182—185°C.

BEISPIEL 42

*2-(4-Amino-5-brom-2-chlor-phenoxy-methylen)-4-isopropyl-morpholin-hydrochlorid*

Hergestellt aus 2-(4-Amino-2-chlor-phenoxy-methylen)-4-isopropyl-morpholin-hydrochlorid und Brom analog Beispiel 5.

Schaum, Strukturbeweis durch NMR-Spektrum. Lösungsmittel $CDCl_3/CD_3OD$:

| | | | |
|---|---|---|---|
| 7,0 und 6,7 ppm | 2 | Singulette | (2 arom. C*H*) |
| 4,4 ppm | | Singulett | (2 austauschbare H) |
| 3,4 — 4,0 ppm | | Multiplett | (5 H neben O) |
| 2,0 — 3,0 ppm | | Multiplett | (5 H neben N) |
| 1,0 und 1,1 ppm | | Dublett | (6 H Isopropyl) |

BEISPIEL 43

*2-(4-Amino-5-brom-2-chlor-phenoxy-methylen)-morpholin-hydrochlorid*

Hergestellt aus 2-(4-Amino-2-chlor-phenoxy-methylen)-morpholin-dihydrochlorid und Brom analog Beispiel 5.

Schmelzpunkt: 220—223°C.

BEISPIEL 44

*2-(2-Amino-3-brom-5-methyl-phenoxy-methylen)-4-benzyl-morpholin-hydrochlorid*

Hergestellt aus 2-(2-Amino-5-methyl-phenoxy-methylen)-4-benzyl-morpholin-hydrochlorid und Brom analog Beispiel 5.

Schmelzpunkt: 223—226°C.

BEISPIEL 45

*2-(2-Amino-3-brom-5-methyl-phenoxy-methylen)-morpholin-hydrochlorid*

Hergestellt aus 2-(2-Amino-5-methyl-phenoxy-methylen)-morpholin-hydrochlorid und Brom analog Beispiel 5.

Schmelzpunkt: 225—227°C.

BEISPIEL 46

*2-(2-Amino-3-brom-5-methyl-phenoxy-methylen)-4-isopropyl-morpholin-hydrochlorid*

Hergestellt aus 2-(2-Amino-5-methyl-phenoxy-methylen)-4-isopropyl-morpholin und Brom analog Beispiel 5.

Schmelzpunkt: 205—207°C.

BEISPIEL 47

*2-(4-Amino-3-brom-5-methyl-phenoxy-methylen)-4-benzyl-morpholin-dihydrochlorid*

Hergestellt aus 2-(4-Amino-5-methyl-phenoxy-methylen)-4-benzyl-morpholin-dihydrochlorid und Brom analog Beispiel 5.

Schmelzpunkt: 241—243°C (Zers.).

BEISPIEL 48

*2-(2-Amino-3,5-dibrom-4-nitro-phenoxy-methylen)-4-benzyl-morpholin-dihydrochlorid*

Hergestellt aus 2-(2-Amino-4-nitro-phenoxy-methylen)-4-benzyl-morpholin-dihydrochlorid und Brom analog Beispiel 5.

Schmelzpunkt: 122—125°C.

BEISPIEL 49

*2-(2-Amino-3,5-dibrom-4-nitro-phenoxy-methylen)-4-isopropyl-morpholin-dihydrochlorid*

Hergestellt aus 2-(2-Amino-4-nitro-phenoxy-methylen)-4-isopropyl-morpholin-dihydrochlorid und Brom analog Beispiel 5.

Schmelzpunkt: 110—114°C.

BEISPIEL 50

*2-(2-Amino-4-chloro-phenoxy-methylen)-morpholin-dihydrochlorid*

Hergestellt aus 2-(2-Amino-4-chlor-phenoxy-methylen)-4-carbäthoxy-morpholin und Natriumhydroxid analog Beispiel 7.

Schmelzpunkt: 247—251°C.

BEISPIEL A

*Tabletten zu 50 mg 2-(2-Amino-3,5-dibrom-phenoxy-methylen)-morpholin-hydrochlorid*

Zusammensetzung:

1 Tablette enthält:

| | |
|---|---|
| Wirksubstanz | 50,0 mg |
| $CaHPO_4$ | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

*Beschreibung:*

Tablettengewicht: 200 mg

Durchmesser: 8 mm, rund, biplan, beidseitige Facette, einseitige Teilkerbe

*Herstellung:*

Wirkstoff, $CaHPO_4$, Milchzucker und Maisstärke werden mit der wäßrigen PVP-Lösung gleichmäßig befeuchtet. Dann wird die Masse durch 2 mm-Maschenweite gesiebt, im Umlufttrockenschrank bei 50°C getrocknet und erneut gesiebt (1,5 mm-Maschenweite). Nach Zumischen des Schmiermittels wird die Mischung auf einer Tablettiermaschine verpreßt.

BEISPIEL B

*Dragées zu 20 mg 2-(2-Amino-3,5-dibrom-phenoxy-methylen)-morpholin-hydrochlorid*

1 Dragéekern enthält:

| | |
|---|---|
| Wirksubstanz | 20,0 mg |
| $CaHPO_4$ | 35,0 mg |
| Milchzucker | 16,8 mg |
| Maisstärke | 16,0 mg |
| Polyvinylpyrrolidon | 1,5 mg |
| Magnesiumstearat | 0,7 mg |
| | 90,0 mg |

*Beschreibung:*

Kerndurchmesser: 6 mm, rund, bikonvex, 5 mm Wölbungsradius

*Kernherstellung:*

Erfolgt analog Beispiel A.

*Dragierung:*

Erfolgt im Dragierkessel mit einer gebräuchlichen Zucker-Dragiersuspension und anschließender Polierung.

Dragéegewicht: 130 mg

BEISPIEL C

*Kapseln zu 25 mg 2-(2-Amino-3,5-dibrom-phenoxy-methylen)-morpholin-hydrochlorid*

1 Kapsel enthält:

| | |
|---|---|
| Wirksubstanz | 25,0 mg |
| Maisstärke getr. | 72,0 mg |
| Milchzucker pulv. | 50,0 mg |
| Magnesiumstearat | 3,0 mg |
| | 150,0 mg |

*Herstellung:*

Wirkstoff und Hilfsstoffe werden durch ein Sieb von 0,75 mm-Maschenweite gegeben und anschließend gut miteinander vermischt.

Abfüllung in Hartgelatine-Kapseln der Größe 4.

Das Füllgewicht wird laufend überprüft.

Kapselfüllung: 150 mg.

BEISPIEL D

*Salt mit 25 mg 2-(2-Amino-3,5-dibrom-phenoxy-methylen)-morpholin-hydrochlorid pro 15 ml (1 Eßlöffel)*

100 ml Saft enthalten:

| | | | |
|---|---|---|---|
| Wirksubstanz | | 0,1667 | g |
| Carboxymethylcellulose | | 0,1 | g |
| p-Hydroxybenzoesäuremethylester | | 0,05 | g |
| p-Hydroxybenzoesäurepropylester | | 0,01 | g |
| Rohrzucker | | 10,0 | g |
| Glycerin | | 5,0 | g |
| Sorbitlösung 70% | | 20,0 | g |
| Aroma | | 0,3 | g |
| Wasser dest. | ad | 100,0 | ml |

*Herstellungsverfahren:*

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und gelöst. Nach Zugabe und Lösung des Zuckers, der Sorbitlösung und des Aromas wird zur Entlüftung des Saftes unter Rühren evakuiert.

*15 ml Saft enthalten 25 mg Wirksubstanz*

$R_4$ ein Wasserstoffatom, eine Alkylgruppe mit 1 bis 4 Kohlenstoffatomen, eine Carbalkoxygruppe mit insgesamt 2 bis 4 Kohlenstoffatomen, die Phenyl- oder Benzylgruppe bedeuten, und deren physiologisch verträgliche Säureadditionssalze.

3. Neue 2-Amino- und 4-Amino-phenoxymethyl-2-morpholinderivate der Formel I nach Anspruch 1, in der einer der Reste $R_1$ oder $R_2$ in 3- oder 5-Stellung ein Bromatom und der andere der Reste $R_1$ oder $R_2$ in 3- oder 5-Stellung ein Wasserstoff- oder Bromatom,
$R_3$ ein Wasserstoffatom und
$R_4$ ein Wasserstoffatom, eine Alkylgruppe mit 1 bis 4 Kohlenstoffatomen oder die Carbäthoxygruppe bedeuten, und deren physiologisch verträgliche Säureadditionssalze.

4. 2-(2-Amino-3,5-dibrom-phenoxy-methylen)-morpholin und dessen Säureadditionssalze.

5. 2-(4-Amino-3,5-dibrom-phenoxy-methylen)-4-isopropyl-morpholin und dessen Säureadditionssalze.

6. 2-(4-Amino-3,5-dibrom-phenoxy-methylen)-morpholin und dessen Säureadditionssalze.

7. 2-(2-Amino-3,5-dibrom-phenoxy-methylen)-4-tert.butyl-morpholin und dessen Säureadditionssalze.

8. Arzneimittel, enthaltend eine Verbindung nach Anspruch 1 bis 7 neben einem oder mehreren inerten Trägerstoffen oder Verdünnungsmitteln.

9. Verwendung einer Verbindung nach Anspruch 1 bis 7 zur Herstellung eines Arzneimittels auf nichtchemischem Wege zur Behandlung von Depressionen und von Angstzuständen.

10. Verfahren zur Herstellung von Aminophenoxymethyl-2-morpholin-derivaten nach Anspruch 1 bis 7, dadurch gekennzeichnet, daß

a) eine Nitroverbindung der Formel

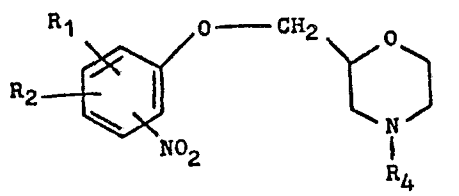

, (II)

in der
$R_1$, $R_2$ und $R_4$ wie eingangs definiert sind, reduziert wird oder

b) von einer Verbindung der Formel

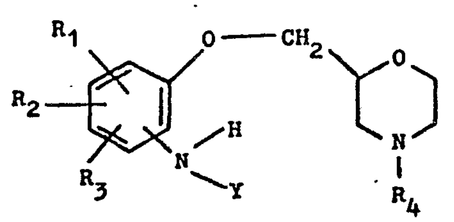

, (III)

in der
$R_1$ bis $R_4$ wie eingangs definiert sind und
Y eine Schutzgruppe für eine Aminogruppe wie die Benzyl-, Formyl-, Acetyl-, Benzoyl- oder Carbäthoxygruppe darstellt, ein Schutzrest hydrolytisch oder hydrogenolytisch abgespalten wird und gewünschtenfalls ein Verhindung der allgemeinen Formel I, in der $R_3$ und $R_4$ wie eingangs definiert sind und $R_1$ und/oder $R_2$ ein Wasserstoffatom darstellen, anschließend mittels Halogenierung in eine entsprechende Chlor- oder Bromverbindung der Formel I übergeführt wird,
und/oder eine Verbindung der Formel I, in der $R_4$ einen Aralkylrest darstellt, anschließend mittels eines Halogenameisensäureesters in eine Verbindung der Formel I, in der $R_4$ eine Carbalkoxygruppe darstellt, übergeführt wird,
und/oder eine Verbindung der Formel I, in der $R_4$ eine Carbalkoxygruppe darstellt, anschließend mittels Hydrolyse in eine Verbindung der Formel I, in der $R_4$ ein Wasserstoffatom darstellt, übergeführt wird,
und/oder eine Verbindung der Formel I in ihre physiologisch verträglichen Säureadditionssalze mit anorganischen und organischen Säuren übergeführt wird.

BEISPIEL E

*Ampullen zu 10 mg 2-(2-Amino-3,5-dibrom-phenoxy-methylen)-morpholin-hydrochlorid*

1 Ampulle enthält:

| | | |
|---|---|---|
| Wirksubstanz | | 10,0 mg |
| Zitronensäure | | 1,0 mg |
| $Na_2HPO_2 \cdot 2H_2O$ | | 3,5 mg |
| Natriumchlorid | | 6,5 mg |
| Aqua dest | ad | 1,0 ml |

*Herstellungsbeschreibung:*

Zu einem geeichten Ansatzgefäß aus indifferentem Material wird die Hauptmenge des $N_2$-gesättigten Ampullenwassers vorgelegt und nacheinander die Puffersubstanzen, die Wirksubstanz und das Natriumchlorid unter Rühren und ständiger $N_2$-Begasung gelöst. Danach wird mit dem restlichen Ampullenwasser bis zur Eichmarke aufgefüllt und durch Membranfilter sterilfiltriert. Die Abfüllung erfolgt unter Vor- und Nachbegasung mit $N_2$ in gereinigte und sterilisierte 1 ml-Ampullen aus braunem Glas.

BEISPIEL F

*Suppositorien zu 50 mg 2-(2-Amino-3,5-dibrom-phenoxy-methylen)-morpholin-hydrochlorid*

1 Zäpfchen enthält:

| | |
|---|---|
| Wirksubstanz | 0,050 g |
| Zäpfchenmasse (z.B. Witepsol H 19 und | 1,238 g |
| Witepsol E 45) | 0,412 g |
| | 1,700 g |

*Herstellungsbeschreibung:*

In die aus Suppositorienmasse bereitete und auf ca. 40°C temperierte Schmelze wird der gemahlene Wirkstoff unter Rühren eingearbeitet und gleichmäßig verteilt. Die fertige Suppositorienmasse wird bei laufendem Rührwerk in vorgekühlte Formen gegossen; nach dem völligen Erstarren werden die Zäpfchen der Form entnommen.

Zäpfchengewicht: 1,7 g.

**Patentansprüche:**

1. Aminophenoxymethyl-2-morpholinderivate der Formel

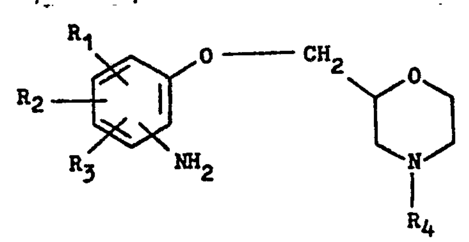

, (I)

in der

$R_1$ ein Wasserstoff- oder Halogenatom oder die Methylgruppe,
$R_2$ ein Wasserstoff- oder Halogenatom,
$R_3$ ein Wasserstoffatom oder die Nitrogruppe und
$R_4$ ein Wasserstoffatom, eine niedere Alkylgruppe, eine Carbalkoxygruppe, die Phenyl- oder Benzylgruppe bedeuten, und deren physiologisch verträgliche Säureadditionssalze.

2. Aminophenoxymethyl-2-morpholinderivate der Formel I nach Anspruch 1, in der
$R_1$ ein Wasserstoff-, Fluor-, Chlor- oder Bromatom oder die Methylgruppe,
$R_2$ ein Wasserstoff-, Fluor-, Chlor- oder Bromatom,
$R_3$ ein Wasserstoffatom oder die Nitrogruppe und

**Revendications**

1. Dérivés d'aminophénoxyméthyl-2-morpholine de formule:

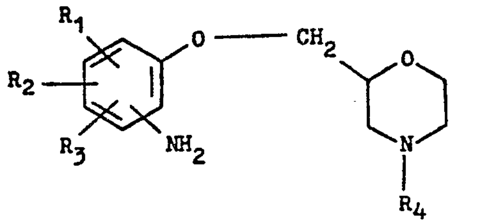

, (I)

dans laquelle:
— $R_1$ représente un atome d'hydrogène ou d'halogène ou le groupe méthyle,
— $R_2$ représente un atome d'hydrogène ou d'halogène,
— $R_3$ représente un atome d'hydrogène ou le groupe nitro et
— $R_4$ représente un atome d'hydrogène, un groupe alcoyle inférieur, un groupe carbalcoxy, le groupe phényle ou benzyle, et leurs sels d'addition avec des acides physiologiquement supportables.

2. Dérivés d'aminophénoxyméthyl-2-morpholine de formule I selon la revendication 1, dans laquelle:
— $R_1$ représente un atome d'hydrogène, de fluor, de chlore ou de brome ou le groupe méthyle,
— $R_2$ représente un atome d'hydrogène, de fluor, de chlore ou de brome,
— $R_3$ représente un atome d'hydrogène ou le groupe nitro et
— $R_4$ représente un atome d'hydrogène, un groupe alcoyle comprenant 1 à 4 atomes de carbone, un groupe carbalcoxy avec en tout 2 à 4 atomes de carbone, le groupe phényle ou benzyle, et leurs sels d'addition avec des acides physiologiquement supportables.

3. Nouveaux dérivés de 1-amino et 4-amino-phénoxyméthyl-2-morpholine de formule I selon la revendication 1, dans laquelle l'un des radicaux $R_1$ ou $R_2$ en position 3 ou 5 représente un atome de brome et l'autre des radicaux $R_1$ ou $R_2$ en position 3 ou 5 représente un atome d'hydrogéne ou de brome,
— $R_3$ représente un atome d'hydrogène et
— $R_4$ représente un atome d'hydrogène, un groupe alcoyle comprenant 1 à 4 atomes de carbone ou le groupe carbéthoxy, et leurs sels d'addition avec des acides physiologiquement supportables.

4. La 2-(2-amino-3,5-dibromo-phénoxy-méthylène)-morpholine et ses sels d'addition avec des acides.

5. La 2-(4-amino-3,5-dibromo-phénoxy-méthylène)-4-isopropyl-morpholine et ses sels d'addition avec des acides.

6. La 2-(4-amino-3,5-dibromo-phénoxy-méthylène)-morpholine et ses sels d'addition avec des acides.

7. La 2-(2-amino-3,5-dibromo-phénoxy-méthylène)-4-tert.butyl-morpholine et ses sels d'addition avec des acides.

8. Médicament renfermant un composé selon l'une des revendications 1 à 7 en présence d'un ou de plusieurs excipients inertes ou d'agents de dilution.

9. Utilisation d'un composé selon l'une des revendications l à 7 pour préparer, selon une voie non chimique, un médicament pour le traitement des dépressions et des états d'anxiété.

10. Procédé de préparation des dérivés d'aminophénoxyméthyl-2-morpholine selon l'une des revendications 1 à 7, caractérisé en ce que:

a) on réduit un composé nitré de formule:

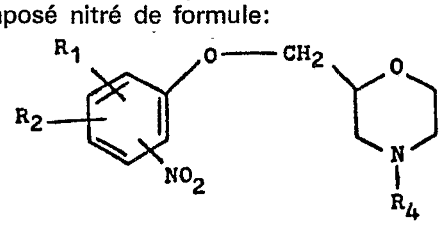

, (II)

dans laquelle:
— $R_1$, $R_2$ et $R_4$ sont définis comme précédemment, ou

b) à partir d'un composé de formule:

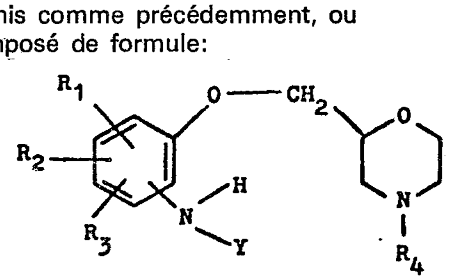

, (III)

dans laquelle:
— $R_1$ à $R_4$ sont définis comme précédemment et
— Y représente un groupe protecteur pour un groupe amino tel que le groupe benzyle, formyle, acétyle, benzoyle ou carbéthoxy,
on élimine hydrolytiquement ou hydrogénolytiquement un groupe protecteur et si on le désire on transforme un composé de formule I dans laquelle $R_3$ et $R_4$ sont définis comme précédemment et $R_1$ et/ou $R_2$ représentent un atome d'hydrogène, subséquemment au moyen d'une halogénation en un composé chloré ou bromé correspondant de formule I,
et/ou on transforme un composé de formule I dans laquelle $R_4$ représente un radical aralcoyle, subséquemment au moyen d'un ester halogénoformique, en un composé de formule I dans laquelle $R_4$ représente un groupe carbalcoxy,
et/ou on transforme un composé de formule I dans laquelle $R_4$ représente un groupe carbalcoxy, subséquemment au moyen d'une hydrolyse en un composé de formule I dans laquelle $R_4$ représente un atome d'hydrogène,
et/ou on transforme un composé de formule I en ses sels d'addition physiologiquement supportables avec des acides minéraux et organiques.

**Claims**

1. Aminophenoxymethyl-2-morpholine derivatives of formula

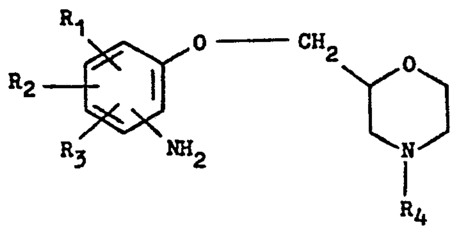

,(I)

in which
$R_1$ represents a hydrogen or halogen atom or the methyl group,
$R_2$ represents a hydrogen or halogen atom,
$R_3$ represents a hydrogen atom or the nitro group and
$R_4$ represents a hydrogen atom, a lower alkyl group, a carbalkoxy group, the phenyl or benzyl group, and their physiologically compatible acid addition salts.

2. Aminophenoxymethyl-2-morpholine derivatives of formula I according to claim 1 in which
$R_1$ represents a hydrogen, fluorine, chlorine or bromine atom or the methyl group,
$R_2$ represents a hydrogen, fluorine, chlorine or bromine atom,
$R_3$ represents a hydrogen atom or the nitro group and
$R_4$ represents a hydrogen atom, an alkyl group with 1 to 4 carbon atoms, a carbalkoxy group with altogether 2 to 4 carbon atoms, the phenyl or benzyl group, and their physiologically compatible acid addition salts.

3. Novel 2-amino- and 4-amino-phenoxymethyl-2-morpholine derivatives of formula I according to claim 1 in which one of the radicals $R_1$ or $R_2$ in 3- or 5-position represents a bromine atom and the other of the radicals $R_1$ or $R_2$ in 3- or 5-position represents a hydrogen or bromine atom,
$R_3$ represents a hydrogen atom and
$R_4$ represents a hydrogen atom, an alkyl group with 1 to 4 carbon atoms or the carbethoxy group, and their physiologically compatible acid addition salts.

4. 2-(2-Amino-3,5-dibromo-phenoxy-methylene)-morpholine and its acid solution salts.

5. 2-(4-Amino-3,5-dibromo-phenoxy-methylene)-4-isopropyl-morpholine and its acid addition salts.

6. 2-(4-Amino-3,5-dibromo-phenoxy-methylene)-morpholine and its acid addition salts.

7. 2-(2-Amino-3,5-dibromo-phenoxy-methylene)-4-tert.butyl-morpholine and its acid addition salts.

8. Pharmaceuticals containing a compound according to claims 1 to 7 together with one or more inert carriers or diluting agents.

9. Use of a compound according to claims 1 to 7 for the preparation of a pharmaceutical in a non-chemical way for the treatment of depressions and anxiety conditions.

10. Process for the preparation of aminophenoxymethyl-2-morpholine derivatives according to claims 1 to 7, characterised in that

a) a nitro compound of formula

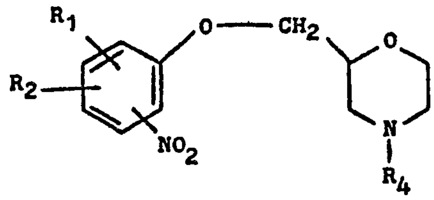

, (II)

in which

$R_1$, $R_2$ and $R_4$ are as defined above is reduced

or

b) a protective radical is separated by hydrolysis or hydrogenolysis from a compound of formula

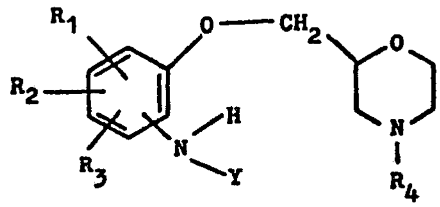

, (III)

in which

$R_1$ to $R_4$ are as defined above and

Y represents a protective group for an amino group such as the benzyl, formyl, acetyl, benzoyl or carbethoxy group and, if desired, a compound of formula I in which $R_3$ and $R_4$ are as defined above and $R_1$ and/or $R_2$ represent a hydrogen atom is subsequently converted by means of a halogenation into a corresponding chlorine or bromine compound of formula I

and/or a compound of formula I in which $R_4$ represents an aralkyl radical is subsequently converted by means of a haloformate into a compound of formula I in which $R_4$ represents a carbalkoxy group

and/or a compound of formula I in which $R_4$ represents a carbalkoxy group is subsequently converted by means of hydrolysis into a compound of formula I in which $R_4$ represents a hydrogen atom

and/or a compound of formula I is converted into its physiologically compatible acid addition salts with inorganic and organic acids.